(19) Europäisches Patentamt
European Patent Office
Office européen des brevets

(11) **EP 1 922 993 B1**

(12) **EUROPÄISCHE PATENTSCHRIFT**

(45) Veröffentlichungstag und Bekanntmachung des Hinweises auf die Patenterteilung:
**07.08.2013 Patentblatt 2013/32**

(51) Int Cl.:
*A61B 5/04* (2006.01)   *A61B 5/042* (2006.01)
*A61B 5/0456* (2006.01)

(21) Anmeldenummer: **07020555.4**

(22) Anmeldetag: **20.10.2007**

(54) **Messeinrichtung zur Erfassung des Myokardzustandes des Herzens**

Measuring device for recording the myocardial status of the heart

Dispositif pour la détermination de l'état du myocarde du coeur

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **18.11.2006 DE 102006054474**

(43) Veröffentlichungstag der Anmeldung:
**21.05.2008 Patentblatt 2008/21**

(73) Patentinhaber: **Biotronik CRM Patent AG**
**6341 Baar (CH)**

(72) Erfinder:
• **Anosov, Oleg, Dr.**
**91054 Erlangen (DE)**
• **Khassanov, Ildar, Dr.**
**91056 Erlangen (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
**WO-A-2005/042090**

• **ALTUNKESER BüLENT B ET AL: "Can P wave parameters obtained from 12-lead surface electrocardiogram be a predictor for atrial fibrillation in patients who have structural heart disease?" ANGIOLOGY, WESTMINSTER PUBLICATIONS, INC. GLEN HEAD, NY, US, Bd. 54, Nr. 4, Juli 2003 (2003-07), Seiten 475-479, XP009096863 ISSN: 0003-3197**

**Beschreibung**

Die Erfindung betrifft ein Verfahren zur Erfassung des Myokardzustandes des Herzens sowie eine Messeinrichtung dieses Verfahrens.

Kardiologische Experimente und Untersuchungen haben zu der Erkenntnis geführt, dass die Änderung des Zustands des Myokards häufig zu einem Wechsel der Richtung der Erregungsausbreitung der kardiologischen Reizsignale im Herzen führt. Dies beruht zum Beispiel auf den unterschiedlichen Geschwindigkeiten der Erregungsausbreitung in gesunden Myokardbereichen einerseits und etwa in von einer Ischämie befallenen Myokardbereichen andererseits. Ein weiterer Grund kann die Änderung der Geometrie der Herzabteilungen bei der Herzinsuffizienz oder Kardiomyopathie, bei Operationswunden oder bei einem Myokardinfarkt liegen. Richtungswechsel der Erregungsausbreitung können ferner durch eine Änderung des Myokardzustandes vor einem Herzflimmern oder auch durch psychologische Stresssituationen und Belastungen auftreten.

Derartige Störungen können grundsätzlich durch komplexe Auswertungen von kardiologischen Reizsignalen erkannt werden, bekanntermaßen sind jedoch hierfür aufwändige Multielektrodenmessungen notwendig, wie sie beispielsweise bei einem EKG vorgenommen werden. Dies ist für die Implementierung entsprechender Messverfahren und Einrichtungen in einem Implantat praktisch nicht realisierbar.

WO-A-2005/042090 offenbart ein implantierbares medizinisches Gerät welches ein intrakardiales Elektrokardiogramm mittels im Atrium und Ventrikel platzierten Elektroden aufnimmt und in der Lage ist, die positive Maximalamplitude des kardialen Signals zu bestimmen. Allerdings kann aus diesen Maximalamplituden nicht auf den Myocardzustand des Herzens geschlossen werden.

Davon ausgehend liegt der Erfindung die Aufgabe zugrunde, ein Verfahren zur Erfassung des Myokardzustandes des Herzens sowie eine entsprechende Messeinrichtung anzugeben, die auf messtechnisch und konstruktiv einfache Weise realisierbar und damit grundsätzlich in einem Implantat implementierbar sind.

Diese Aufgabe wird durch die folgenden Verfahrensschritte in verfahrenstechnischer Hinsicht wie folgt gelöst:

- Einsetzen einer bipolaren kardiologischen Messelektrode in eine Herzabteilung mit einem spitzen Aufsetzwinkel der Messelektrode auf das Myokard von unter 90°,
- Messen eines kardiologischen Reizsignals in aufeinanderfolgenden Herzzyklen,
- Bestimmen der positiven und negativen Maximalamplituden $V_p$ und $V_n$ des Reizsignals aufeinanderfolgender Herzzyklen,
- Ermitteln eines Asymmetriefaktors $\eta$ des Reizsignals aufeinanderfolgender Herzzyklen nach der Beziehung

$$\eta = (V_p - |V_n|)/(V_p + |V_n|)$$

und

- Speicherung des Asymmetriefaktors $\eta$ von aufeinanderfolgenden Herzzyklen zu dessen Analyse.

Wie anhand des Ausführungsbeispiels noch näher erläutert wird, geht die Erfindung von der Erkenntnis aus, dass bei einer an sich unüblichen, nicht senkrecht zur Myokardebene platzierten Messelektrode der Wechsel der Richtung der Erregungsausbreitung zu einer Änderung der Asymmetrie von intrakardialen elektrischen Signalen (im folgenden kurz "IEGM") führt. Dies macht sich die Erfindung zunutze, indem der angegebenen spitze Aufsetzwinkel der Messelektrode von deutlich unter 90° und vorzugsweise etwa 45° gewählt wird. Aus den entsprechenden Reizsignalen kann der angegebene Asymmetriefaktor $\eta$ bestimmt und für die nachfolgende, nicht zum erfindungsgemäßen Verfahren gehörende Diagnostik dessen Myokardzustandes, insbesondere für eine Diagnostik der Myokardischämie und der Herzinsuffizienz gespeichert werden.

Je nach Platzierung der Messelektrode in einer Herzabteilung wird ein bestimmtes IEGM verwendet, so für eine in das Ventrikel eingesetzte Messgeräte die R-Spitze und für eine in das Atrium eingesetzte Messelektrode die P-Spitze des kardiologischen Reizsignals.

Eine entsprechende Messeinrichtung zur Durchführung des vorstehend erörterten Verfahrens ist Gegenstand des Anspruches 1. Diese Messeinrichtung weist somit auf

- eine in eine Herzabteilung (8) mit einem spitzen Aufsetzwinkel (9) auf das Myokard (6) positionierbare Messelektrode (4), sowie
- eine die Messsignale (IEGM) der Messelektrode (4) abnehmende, implantierbare Verarbeitungseinrichtung (2) zur Aufnahme, Erfassung, Weitersendung und/oder Auswertung der Messsignale, welche für einen Herzzyklus aus den kardiologischen Reizsignalen die positive und negative Maximalamplitude $V_p$ und $V_n$ und einen Asymmetriefaktor $\eta$ nach der Beziehung:

$$\eta = (V_p - |V_n|)/(V_p + |V_n|)$$

bestimmt..

Auf der Basis der Analyse des Asymmetriefaktors der IEGM-Signale können also Geräte für die Diagnostik des Myokardzustandes konzipiert werden. Die Einfachheit der Methode und insbesondere die geringen hierfür notwendigen Ausgangssignale in Form der positiven und negativen Maximalamplituden eines Reizsignals erlauben es, die entsprechenden Verfahren in modernen implantierbaren Geräten anzuwenden. Vorzugsweise ist also ein Herzschrittmacher oder Defibrillator mit einer integrierten Auswerteeinrichtung zur Ermittlung des Asymmetriefaktors $\eta$ versehen.

Alternativ oder zusätzlich dazu kann die Verarbeitungseinrichtung der Messeinrichtung einen Sender zur Weitersendung der ermittelten Messsignale an eine externe Auswertestation aufweisen, in der der Asymmetriefaktor $\eta$ dann ermittelbar ist.

Weitere Merkmale, Einzelheiten und Vorteile der Erfindung sind der nachfolgenden Beschreibung entnehmbar, in der ein Ausführungsbeispiel des erfindungsgemäßen Verfahrens und eine entsprechende Messeinrichtung näher erläutert werden. Es zeigen:

Fig. 1    eine schematische Ansicht eines Patiententhorax mit implantiertem Herzschrittmacher und Messelektrode im rechten Ventrikel,

Fig. 2    eine schematische vergrößerte Ansicht der im Myokard verankerten Messelektrode,

Fig. 3    ein Diagramm analytisch errechneter IEGM-Signale für verschiedene Richtungen der Erregungsausbreitung,

Fig. 4 und 5    IEGM-Signale mit unterschiedlichen Asymmetrien, und

Fig. 6    ein zeitliches Diagramm des Blutdrucks (a) und des entsprechenden IEGM-Asymmetriefaktors $\eta$ (b).

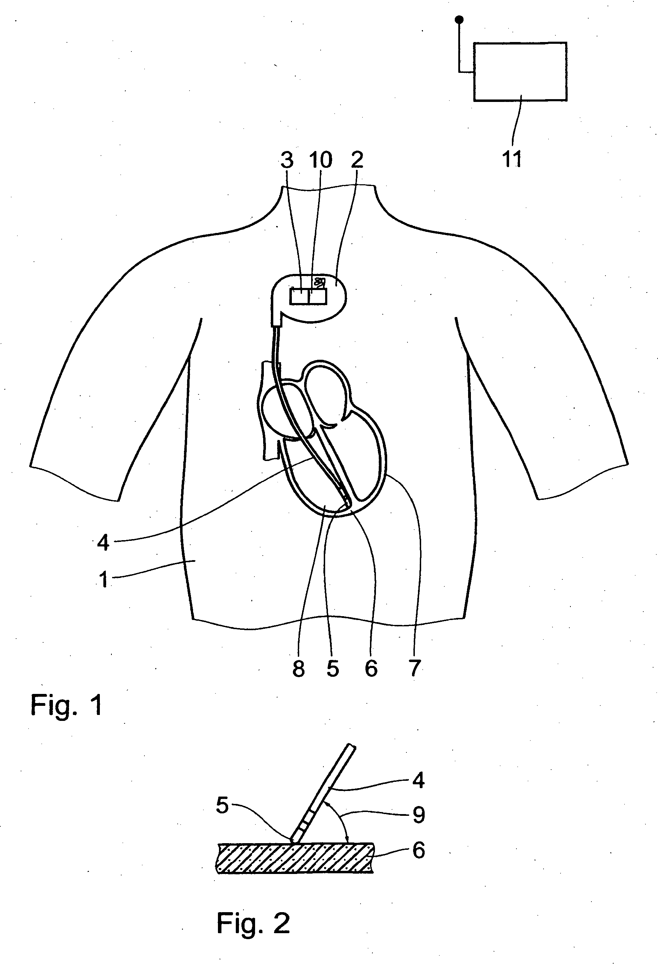

Fig. 1 zeigt einen in den Thorax 1 eines Herzpatienten eingepflanzten Herzschrittmacher 2, in den eine Auswerteeinrichtung 3 für den noch näher zu erläuternden, oben erwähnten Asymmetriefaktor $\eta$ integriert ist. Diese Auswerteeinrichtung 3 verarbeitet die Messsignale der bipolaren Messelektrode 4, die mit ihrer Elektrodenspitze 5 im Myokard 6 des Herzens 7 im Bereich des rechten Ventrikels 8 verankert ist. Wie Fig. 2 zeigt, ist die Messelektrode 4 dabei in einem spitzen Winkel 9 von etwa 45° zur Ebene des Myokards 6 geneigt.

Die gemessenen Signale der Messelektrode 4 oder von der Auswerteeinrichtung 3 generierte Daten können über den Sender 10 an eine externe Auswertestation 11 übermittelt werden, wo sie beispielsweise im Zuge eines sogenannten "Home Monitorings" weiterverarbeitet und genutzt werden.

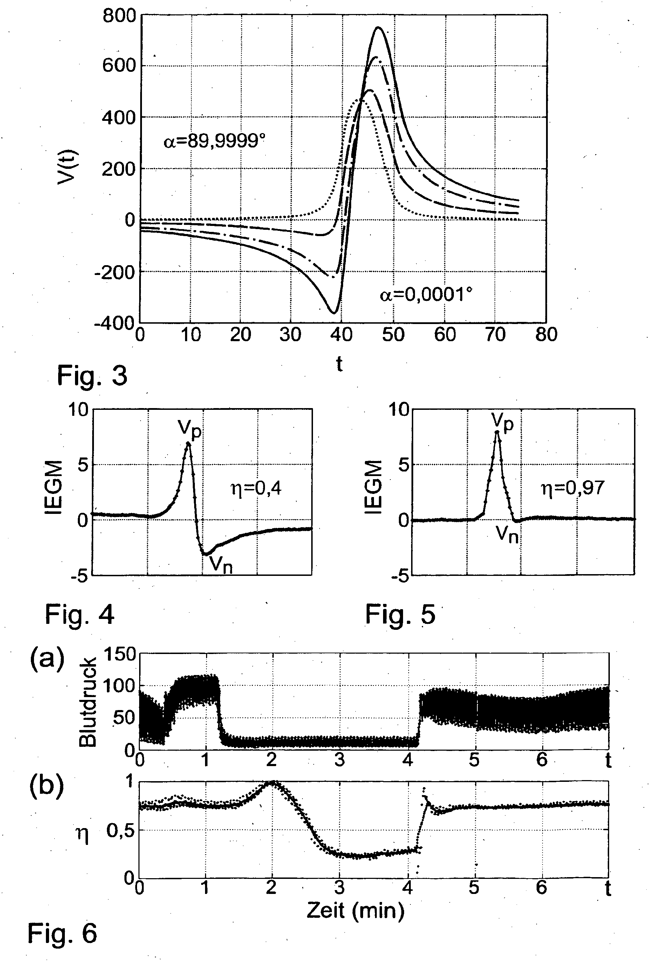

Analytische Auswertungen von medizinischen Versuchen haben gezeigt, dass bei einer nicht senkrechten Positionierung der Messelektrode 4 zur Ebene des Myokards 6 ein Wechsel der Richtung der Erregungsausbreitung zu einer Änderung der Asymmetrie von interkardialen elektrischen Signalen führt. In Fig. 3 sind für

einige Winkel $\alpha$ zwischen der Richtung der Erregungsausbreitung und der Projektion der Messelektrode 4 auf die Myokardfläche errechnete IEGM-Signale dargestellt.

Fig. 4 und 5 zeigen IEGM-Signale in Form von R-Spitzen der gemäß Fig. 1 in das rechte Ventrikel eingesetzten Messelektrode 4. Es sind die Amplitude $V_p$ der positiven Spitze und die Amplitude $V_n$ der negativen Spitze des jeweiligen Signals aufgetragen. Gemäß der Beziehung

$$\eta = (V_p - |V_n|)/(V_p + |V_n|)$$

berechnet sich der Asymmetriefaktor $\eta$ bei Fig. 4 zu $\eta$ = 0.4 und in Fig. 5 zu $\eta$ = 0.97.

Die Signifikanz des Asymmetriefaktors $\eta$ für eine pathologische Situation des Herzens lässt sich anhand von Fig. 6 darlegen. Das Diagramm gemäß (a) zeigt den Blutdruck in der Koronararterie während eine Ischämie, in einem Zeitraum zwischen etwa t = 1 Minute und t = 4 Minuten aufgetreten ist.

Mit kurzer Verzögerung durchläuft der in Teilfigur (b) aufgezeichnete Asymmetriefaktor $\eta$ ein Maximum bei t = 2 Minuten, um dann deutlich auf einen Wert von etwa einem Drittel des ursprünglichen Asymmetriefaktors abzusinken.

Die Signifikanz der Änderung des Asymmetriefaktors für das Vorliegen einer Ischämie ist deutlich.

**Patentansprüche**

1. Implantierbare Messeinrichtung zur Erfassung des Myocardzustandes des Herzens, bestehend aus einer bipolaren kardiologischen Messelektrode (4), welche in eine Herzabteilung (8) mit einem spitzen Aufsetzwinkel (9) der Messelektrode (4) auf das Myokard (6) von unter 90° aufgesetzt ist und einer die Messsignale der Messelektrode (4) abnehmende Verarbeitungseinrichtung (2) zur Verarbeitung der erfassten kardiologischen Reizsignale, **dadurch gekennzeichnet, dass** die Verarbeitungseinrichtung (2) für einen Herzzyklus aus den kardiologischen Reizsignalen die positive und negative Maximalamplitude $V_p$ und $V_n$ und einen Asymmetriefaktor $\eta$ nach der Beziehung $\eta = (V_p - |V_n|)/(V_p + |V_n|)$ bestimmt.

2. Implantierbare Messeinrichtung nach Anspruch 1, **dadurch gekennzeichnet, dass** die kardiologischen Reizsignale im Atrium erfasst werden und die P-Spitze des Herzzyklus beinhalten.

3. Implantierbare Messeinrichtung nach Anspruch 1, **dadurch gekennzeichnet, dass** die kardiologischen Reizsignale im Ventrikel erfasst werden und die R-Spitze des Herzzyklus beinhalten.

**4.** Implantierbare Messeinrichtung nach einem der vorgenannten Ansprüche, **gekennzeichnet durch** Mittel zur Speicherung des Asymmetriefaktors η von aufeinander folgenden Herzzyklen.

**5.** Implantierbare Messeinrichtung nach einem der vorgenannten Ansprüche, **dadurch gekennzeichnet, dass** die Verarbeitungseinrichtung einen Sender (10) zur Weitersendung der erfassten kardiologischen Reizsignale und/oder des Asymmetriefaktors η an eine externe Auswertestation (11) aufweist.

**6.** Implantierbare Messeinrichtung nach einem der vorgenannten Ansprüche, **dadurch gekennzeichnet, dass** die Verarbeitungseinrichtung durch einen Herzschrittmacher oder Defibrillator gebildet ist.

## Claims

**1.** An implantable measuring device for detecting the myocardial state of the heart, comprising a bipolar cardiological measuring electrode (4), which is placed in a heart compartment (8) onto the myocardium (6) at an acute placement angle (9) of the measuring electrode (4) of less than 90°, and a processing device (2), which picks up the measurement signals of the measuring electrode (4), for processing the detected cardiological stimulation signals, **characterized in that** the processing device (2) determines the positive and negative maximum amplitudes $V_p$ and $V_n$ and an asymmetry factor η according to the relationship $\eta = (V_p - |V_n|)/(V_p + |V_n|)$ for a cardiac cycle based on the cardiological stimulation signals.

**2.** The implantable measuring device according to claim 1, **characterized in that** the cardiological stimulation signals are detected in the atrium and include the P peak of the cardiac cycle.

**3.** The implantable measuring device according to claim 1, **characterized in that** the cardiological stimulation signals are detected in the ventricle and include the R peak of the cardiac cycle.

**4.** An implantable measuring device according to any one of the preceding claims, **characterized by** means for storing the asymmetry factor η of consecutive cardiac cycles.

**5.** An implantable measuring device according to any one of the preceding claims, **characterized in that** the processing device comprises a transmitter (10) for forwarding the detected cardiological stimulation signals and/or the asymmetry factor η to an external evaluation station (11).

**6.** An implantable measuring device according to any one of the preceding claims, **characterized in that** the processing device is formed by a cardiac pacemaker or defibrillator.

## Revendications

**1.** Dispositif de mesure implantable pour la détection de l'état du myocarde d'un coeur, constitué d'une électrode de mesure cardiologique bipolaire (4) disposée dans un compartiment du coeur (8), avec un angle d'appui aigu (9) de moins de 90° de l'électrode de mesure (4) sur le myocarde (6), et d'un dispositif de traitement (2) recevant les signaux de mesure de l'électrode de mesure (4), pour le traitement des signaux d'excitation cardiologiques détectés, **caractérisé en ce que** le dispositif de traitement (2) détermine, pour un cycle cardiaque, l'amplitude maximale négative et positive Vp et Vn et un facteur d'asymétrie η selon le rapport $\eta = (Vp - |Vn|)/(Vp + |Vn|)$, à partir des signaux d'excitation cardiologiques.

**2.** Dispositif de mesure implantable selon la revendication 1, **caractérisé en ce que** les signaux d'excitation cardiologiques sont détectés dans l'atrium et contiennent le pic P du cycle cardiaque.

**3.** Dispositif de mesure implantable selon la revendication 1, **caractérisé en ce que** les signaux d'excitation cardiologiques sont détectés dans le ventricule et contiennent le pic R du cycle cardiaque.

**4.** Dispositif de mesure implantable selon l'une des revendications précédentes, **caractérisé par** des moyens pour l'enregistrement du facteur d'asymétrie η de cycles cardiaques successifs.

**5.** Dispositif de mesure implantable selon l'une des revendications précédentes, **caractérisé en ce que** le dispositif de traitement comporte un émetteur (10) pour la transmission des signaux d'excitation cardiologiques détectés et/ou du facteur d'asymétrie η à une station d'évaluation externe (11).

**6.** Dispositif de mesure implantable selon l'une des revendications précédentes, **caractérisé en ce que** le dispositif de traitement est composé d'un stimulateur cardiaque ou d'un défibrillateur.

3  10  2

11

4
1

8  5  6  7

**Fig. 1**

5

4

9

6

**Fig. 2**

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005042090 A